# EUROPEAN PATENT APPLICATION

(11) **EP 2 070 943 A1**
(43) Date of publication of application: **17.06.2009**
(21) Application number: 07380362.9
(22) Date of filing: 14.12.2007
(51) Int. Cl.: C07J 1/00

(54) **Process for obtaining 6-Alkylidenandrost-1,4-diene-3one**

(71) Applicant: Crystal Pharma, S.A., 47151 Boecillo (ES)
(72) Inventor: Bermejo González, Francisco, Univer. de Salamanca, 37008 Salamanca (ES); Marcos Escribano, José Andrés, 37007 Salamanca (ES); Gutiérrez Fuentes, Luis Gerardo, Crystal Pharma SA, 47151 Boecillo Valladolid (ES); Iglesias Retuerto, Jesús Miguel, Crystal Pharma SA, 47151 Boecillo Valladolid (ES); Lorente Bonde-Larsen, Antonio, Ragactives, S.L., 47151 Boecillo Valladolid (ES)
(74) Representative: ABG Patentes, S.L.

(57) **Abstract**

6-alkylidenandrost-1,4-dien-3-ones of general formula (I), wherein R is H o alkyl; R₁ y R₂, independently of one another, represent H, OR₃, OC(=O) R₄ or O-(GPH), wherein R₃ is H; C₁-C₆ alkyl or aryl; R₄ is H or C₁-C₆ alkyl,; and GPH is a hydroxyl protecting group; or R₁ and R₂, together with the carbon atom to which they are bonded, form a carbonyl group or equivalent or a cyclic ketal; can be obtained by a process comprising subjecting the corresponding 6-alkyliden-4-androsten-3-one to a dehydrogenation reaction in the 1,2 position in the presence of a quinine, a silylating agent and a strong acid.

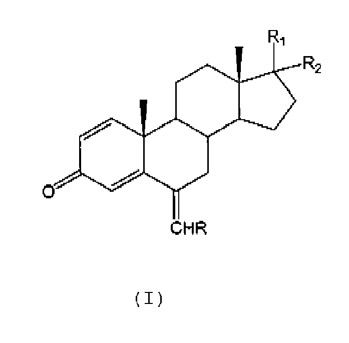

## Description

### FIELD OF THE INVENTION

The invention relates to a process for dehydrogenating steroids, particularly 6-alkylidene-4-androsten-3-one derivatives, for the purpose of obtaining corresponding 1,4-diene derivatives by means of forming an unsaturation in the 1,2 position of the steroid.

### BACKGROUND OF THE INVENTION

Steroids inhibiting estrogen activity are known which are useful in the treatment of cancers associated to hormonal activity, e.g., breast cancer, ovarian cancer, etc. Said steroids include exemestane (6-methylenandrost-1,4-dien-3,17-dione), first described in British patent GB 2177700.

Said British patent describes different processes for introducing an unsaturation in the 1,2 position of the starting steroid (6-methylene-4-androsten-3,17-dione) for the purpose of obtaining the corresponding 6-methylene-1,4-androsten-3,17-dione derivatives, using different dehydrogenating agents, such as selenium dioxide (SeO₂) or 2,3-dichloro-5,6-dicyanobenzoquinone (DDQ) under dioxane reflux. The use of SeO₂ not only provides low yields (40%) but is also expensive and generates contamination problems due to its toxicity, therefore it is not applicable at an industrial level. For its part, the use of DDQ allows obtaining moderate yields (50%) of a product that needs to be purified by chromatography.

DDQ is one of the dehydrogenating agents that is most used to introduce an unsaturation in the 1,2 position of a steroid or of an azasteroid. The dehydrogenation mechanism and conditions using DDQ as a dehydrogenating agent have been analyzed in J. Chem. Soc. (C), (1967), pages 1720-1730. Said article shows that the dehydrogenation of 4-androsten-3,17-dione mainly gives rise to the Δ^{1,4} derivative accompanied by variable amounts of Δ^{1,6} and Δ^{1,4,6} derivatives, depending on the reaction conditions (solvents, temperatures and presence of weak acids as catalysts).

The use of DDQ and dioxane is described, as well as in the previously commented patent GB 2177700, in European patent EP 307135 to transform 17β-hydroxy-6-methylene-4-androsten-3-one into the corresponding 1,4-diene derivative (17βhydroxyl-6-methylenandrost-1,4-diene-3-one), also with a moderate yield (50%) and purification problems. However, the attempts made by the inventors to reproduce this process both in the obtaining of 17β-hydroxy-6-methylenandrost-1,4-diene-3-one and the obtaining of 6-methylenandrost-1,4-diene-3,17-dione from the corresponding 6-methylene-4-androsten derivatives, have not given rise to the desired product (see Examples 5 and 6 of this description), but to the production of a complex mixture of secondary products, detecting only traces of the desired product by high performance liquid chromatography (HPLC).

Chinese patent CN 1453288 describes how to obtain exemestane by dehydrogenation of 6-methylene-4-androsten-3,17-dione using DDQ and an weak organic acid such as benzoic acid as a catalyst. Nevertheless, the attempts made by the inventors to reproduce this process have given rise to moderate yields up' to 14% of the desired product, accompanied by a number of by-products which force to carry out a chromatographic purification, and hence it complicates its industrial application much (see Example 7 of this description).

The use of DDQ as an azasteroid dehydrogenating agent has also been described. The introduction of an unsaturation in the 1,2 position of a 3-oxo-4-azasteroid by means of using DDQ and a silylating agent, such as bistrimethylsilyltrifluoroacetamide (BSTFA) has specifically been described (EP 298652). The incorporation of trifluoromethanesulfonic acid as a catalyst for the dehydrogenation of 3-oxo-4-azasteroids to a combination of DDQ and BSTFA improves the yield of the dehydrogenation reaction EP 748221). However, the mentioned azasteroids are amine derivatives, less reactive than ketones since they are less enolizable, and, on the other hand, the presence of a nitrogen atom in position 4 of the ring A and the absence of other carbonyl groups in the molecule automatically lead to the formation of the double bond in the 1,2 position thus the described conditions are applicable to this kind of steroids (3-oxo-4-aza-steroids) but they do not seem suitable for the androsten-3-one derivatives.

Other dehydrogenating agents which can introduce an unsaturation in the 1,2 position of a steroid are also known. By way of illustration, Chinese patent 1491957 describes the dehydrogenation of 6-methylene-4-androsten-3,17-dione to obtain exemestane using iodoxybenzoic acid (IBX), a oxidizing agent described as potentially explosive, which limits its application at an industrial level.

Other known processes for introducing a Δ¹ double bond in steroids is based on the introduction of leaving groups (e.g., Br, I, etc.) in position 2 of the steroid, which leaving groups are subsequently eliminated in basic medium. Unfortunately in these cases, the introduction of the leaving group in the desired position is not easy to direct, therefore the leaving group is usually introduced in other positions of the steroid, giving rise to the formation of by-products. An illustrative example of this alternative is included in European patent EP 326340, describing the dehydrogenation in the 1,2 position of a 6-methylene-4-androsten-3,17-dione by means of introducing bromine and a subsequent dehydrobromination as shown in the following scheme:

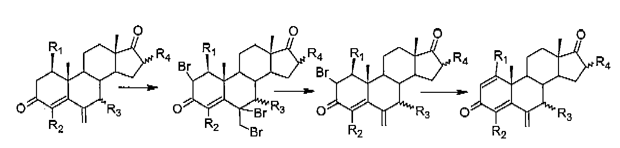

According to this process, bromination not only occurs in position 2 as intended, but also in position 6 (methylene), which forces carrying out 3 steps with a moderate overall yield of the 3 steps (40%).

There is therefore a need to develop an alternative process for obtaining 6-alkylidene-1,4-androsten-2-one derivatives, some of which are of therapeutic interest (e.g., exemestane), which overcomes all or part of the drawbacks associated to the known processes belonging to the state of the art.

### SUMMARY OF THE INVENTION

The invention is faced with the problem of providing an alternative process for obtaining 6-alkylidene-1,4-androsten-3-one derivatives, and particularly exemestane, which overcomes the aforementioned drawbacks existing in the different syntheses of the state of the art.

The solution provided by the invention is based on the fact that the inventors have surprisingly observed that is possible to efficiently obtain 6-alkylidene-1,4-androsten-3-one derivatives and their solvates, by means of dehydrogenating the corresponding 6-alkylidene-4-androsten-3-one derivatives, based on the use of a quinone, a silylating agent and a strong acid, under reaction conditions allowing a regioselective dehydrogenation in the 1,2 position of said 6-alkylidene-4-androsten-3-one derivatives.

A process such as the one provided by the present invention has a number of advantages because the use of reagents that are very harmful for the environment, such as selenium oxide, and the formation of by-products associated to other dehydrogenations are prevented, which favors obtaining the desired product with high yields and enables its isolation with a high purity without having to use expensive purification techniques (e.g., by chromatography). All this contributes to reducing the overall cost of the process, which makes it a commercially interesting process and allows implementation thereof at an industrial level.

Therefore, in one aspect the invention relates to a process for obtaining a 6-alkylidene-1,4-androsten-3-one of general formula (I) (defined below), which comprises reacting a 6-alkylidene-4-androsten-3-one of general formula (II) (defined below) with a quinone in the presence of a silylating agent and of a strong acid acting as a catalyst of the dehydrogenation reaction, and, if desired, converting a compound of general formula (I) thus obtained into another compound of general formula (I) by conventional methods. In a particular embodiment, the compound of general formula (I) obtained is exemestane, an irreversible aromatase inhibitor which can inhibit estrogen activity; it is therefore useful as an antineoplastic agent in the treatment of cancers associated to hormonal activity, e.g., breast cancer, ovarian cancer, etc.

In another aspect the invention relates to a process for obtaining exemestane, which comprises reacting a 6-methylene-4-androsten-3-one of general formula (IIa) (defined below) with a quinone in the presence of a silylating agent and of a strong acid, to obtain a 6-methylene-1,4-androsten-3-one of general formula (Ia) (defined below), and, if needed, converting a compound of general formula (Ia) thus obtained into exemestane.

### DETAILED DESCRIPTION OF THE INVENTION

In one aspect the invention relates to a process, hereinafter process of the invention, for obtaining a 6-alkylidenandrost-1,4-diene-3-one of general formula (I) 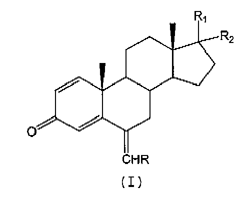 wherein
R is H or C₁-C₆ alkyl;
R₁ and R₂, independently of one another, represent H, OR₃, OC(=O)R₄ or O-(HPG), wherein
R₃ is H, C₁-C₆ alkyl or aryl;
R₄ is H or C₁-C₆ alkyl, and
HPG is a hydroxyl protecting group; or
R₁ and R₂, together with the carbon atom to which they are bonded, form a carbonyl group or equivalent or a cyclic ketal;
and its solvates,
comprising
a) subjecting a compound of general formula (II) 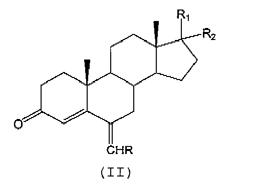 wherein R, R₁ and R₂ have the previously mentioned meanings;
   to a dehydrogenation reaction in the 1,2 position in the presence of a quinone, a silylating agent and a strong acid; and, if desired,
b) converting a compound of general formula (I) into another compound of general formula (I).

The process of the invention comprises subjecting a compound of general formula (II) to a dehydrogenation reaction to introduce an unsaturation in the 1,2 position of the steroid under certain experimental conditions comprising the use of a quinone, a silylating agent and a strong acid, allowing a regioselective dehydrogenation in the 1,2 position of said compounds of general formula (II). In the compound of general formula (II), R is H or C₁-C₆ alkyl; R₁ and R₂, independently of one another, represent H, OR₃, OC(=O)R₄ or O-(HPG), wherein R₃, R, and (HPG) have the previously indicated meanings; or R₁ and R₂, together with the carbon atom to which they are bonded, form a carbonyl group or equivalent or a cyclic ketal.

In a particular embodiment, in the compound of general formula (II), R is H.

In another particular embodiment, in the compound of general formula (II), R is C₁-C₆ alkyl. As used herein, the term "C₁-C₆ alkyl" relates to a radical derived from a linear or branched alkane, with 1 to 6 carbon atoms, methyl, ethyl, propyl, butyl, etc., for example, optionally substituted by one or more substituents independently selected from halogen and C₁-C₆ alkyl.

In another particular embodiment, in the compound of general formula (II), one of R₁ or R₂ is H and the other one is OR₃, wherein R₃ is H.

In another particular embodiment, in the compound of general formula (II), one of R₁ or R₂ is H and the other one is OR₃, wherein R₃ is C₁-C₆ alkyl. Persons skilled in the art will understand that these alkyl groups act as hydroxyl protecting groups.

In another particular embodiment, in the compound of general formula (II), one of R₁ or R₂ is H and the other one is OR₃, wherein R₃ is aryl. As used herein, the term "aryl" relates to a radical derived from an aromatic hydrocarbon, optionally substituted with one or more substituents independently selected from halogen, C₁-C₆ alkyl or N(R₅)(R₆), wherein R₅ and R₆, independently of one another, represent H or C₁-C₆ alkyl, for example, phenyl, benzyl, tolyl, xilyl, etc., non-substituted or substituted with one or more of said substituents. In a specific embodiment, in the compound of general formula (II) one of R₁ or R₂ is H and the other one is OR₃, wherein R₃ is benzyl, optionally substituted.

In another particular embodiment, in the compound of general formula (II), one of R₁ or R₂ is H and the other one is an OC(=O)R₄ acyloxy group, wherein R₄ is H or C₁-C₆ alkyl, for example, O-formyl, O-acetyl, O-propionyl, O-butyryl, etc.

In another particular embodiment, in the compound of general formula (II), one of R₁ or R₂ is H and the other one is O-(HPG), wherein (HPG) is a hydroxyl protecting group. As used in this description, the term "hydroxyl protecting group" includes any group which can protect a hydroxyl group. Illustrative examples of said hydroxyl protecting groups have been described by Green TW et al. in "Protective Groups in Organic Synthesis", 3rd Edition (1999), Ed. John Wiley & Sons (ISBN 0-471-16019-9). Virtually any hydroxyl protecting group can be used for implementing the invention; nevertheless, in a particular embodiment, the hydroxyl protecting group is a group, especially an ester group or an ether group, which can be converted into a hydroxyl group under mild conditions (e.g., acid hydrolysis) for example, alkyl ethers, arylalkyl ethers, enol ethers, silyl ethers, etc.

Illustrative non-limiting examples of said hydroxyl protecting groups include C₁-C₆ alkyl (e.g., methyl, ethyl, n-propyl, iso-propyl, n-butyl, t-butyl, etc.), aryl (e.g., optionally substituted benzyl), silyl, for example, a silyl radical of general formula Si(R₇)(R₈)(R₉) wherein P₇, R₈ and R₉, independently of one another, represent C₁-C₆ alkyl, C₃-C₆ cycloalkyl, aryl, for example, trimethylsilyl, dimethyl-t-butylsilyl, etc.

In another particular embodiment, in the compound of general formula (II), R₁ and R₂ together with the carbon atom to which they are bonded, form a carbonyl group or equivalent or a cyclic ketal. As used herein, the term "equivalent" relates to an imine, oxime, hydrazone group, etc. Likewise, in the sense used in this description, "cyclic ketal" relates to compounds in which the carbon atom in position 17 (to which R₁ and R₂ are bonded) is bonded to an -O(CH2)ₙO- group, wherein n is an integer comprised between 2 and 4, for example, dioxolane, 1,3-dioxane, etc.

Particular embodiments of the invention include the use of compounds of general formula (II) in which:
R is H; or
R₁ is H and R₂ is OH; or
R₁ is H and R₂ is OAc (wherein Ac is C(=O)CH₃); or
R₁ and R₂, together with the carbon atom to which they are bonded, form a carbonyl group (C=O).

The process of the invention comprises reacting the compound of general formula (II), containing a keto group in position 3, with a silylating agent in the presence of a quinone and a strong acid acting as a catalyst of the dehydrogenation reaction. Although it is not desired to be linked to any hypothesis, it is believed that the silylating reagent regioselectively activates the carbonyl group, thus converting it to an enol derivative oriented to the 3,2 position, whereas the strong acid promotes the reaction of said enol derivative with the quinone.

Various silylating agents, particularly silylating agents which can silylate ketones, such as a bistrimethylsilyltrihaloacetamide (wherein halo is a halogen fluorine, chlorine, bromine or iodine), bistrimethylsilylacetamide, hexamethyldisilazane, bistrimethylsilylurea, etc., and mixtures thereof can be used to implement the process of the invention. In a particular preferred embodiment, the silylating agent is bistrimethylsilyltrifluoroacetamide (BSTFA).

Any reactive quinone, understanding by "reactive quinone" a quinone with a reaction potential enough to react with an enol derivative and being removed later making a double bond in the process, for example, 2,3,5,6-tetrachloro-1,4-benzoquinone (chloranil), 2,3-dichloro-5,6-dicyanobenzoquinone (DDQ), etc., and mixtures thereof, preferably chloranil, can be used as quinone. Other quinones with sufficient reaction potential can alternatively be used.

A strong organic or inorganic acid can be used as an acid catalyst. As used herein, a strong acid is an acid having a pKa in aqueous solution equal or less than 1. Illustrative non-limiting examples of strong organic acids include trifluoroacetic acid, p-toluenesulfonic acid, methanesulfonic acid, trifluoromethanesulfonic acid or any other similar organic acid considered to be a strong organic acid. Likewise, illustrative non-limiting examples of strong inorganic acids include sulfuric acid, hydrobromic acid, perchloric acid or any other similar inorganic acid considered to be a strong inorganic acid according to the above definition. One or more organic and/or inorganic acids can be used. In a particular preferred embodiment of the invention, the organic acid is selected from trifluoromethanesulfonic acid, trifluoroacetic acid, p-toluenesulfonic acid and mixtures thereof, and the preferred inorganic acid is selected from sulfuric acid, perchloric acid and mixtures thereof.

The reaction of the compound of general formula (II) with the silylating agent, the quinone and the strong acid is carried out in a suitable solvent, such as an ether, an aliphatic ether (e.g. diisopropylether, etc), a cyclic ether (e.g. tetrahydrofurane (THF), dioxanes, etc) for example, a halogenated solvent, a chlorinated hydrocarbon for example, or preferably an aromatic solvent, benzene, toluene, xylene, etc. for example, at a temperature comprised between room temperature (18-22°C) and the reflux temperature of the solvent used for a time period equal to or greater than 15 minutes, typically comprised between 30 minutes and 48 hours.

Both the temperature of the reaction and the time period necessary for the reaction to take place (reaction duration), will essentially depend on the nature of the quinone used as well as the strength of the (organic or inorganic) acid used. In fact, the more reactive the quinone and the stronger the acid used, the milder the reaction conditions and the shorter the time period necessary for the reaction to take place. In this sense, when the reaction is carried out with chloranil, a very little reactive quinone, the reaction needs stronger conditions, such as the use of a (very) strong acid, e.g., trifluoromethanesulfonic acid, sulfuric acid, or mixtures thereof, and/or higher temperatures allowing the dehydrogenation reaction to take place in a reasonable time period; in contrast, the dehydrogenation takes place with greater regioselectivity, which means a lower amount of impurities or by-products and obtaining higher yields. In these conditions, highly pure products of general formula (I) are achieved with very high yields, typically about 85% or higher.

When more reactive quinones, e.g., DDQ, are used, the reaction can be carried out in milder conditions, such as room temperature and/or less aggressive acid media (see example 3.6).

The presence of the silylating agent is a necessary condition for the reaction to take place effectively; in general, it is needed at least 1, advantageously between 2 and 5, equivalents of silylating agent per equivalent of compound of general formula (II), depending on the conditions used and the nature of the starting product; nevertheless, both the temperature and the time period necessary for the dehydrogenation reaction to take place depend on the reactivity of the quinone used and on the acid character of the catalyst.

The substituents that are possibly found in position 17 of the starting steroid [compound of general formula (II)] can also have an influence. In this sense, the presence of a hydroxyl group in said position 17 can interfere in the necessary amount of silylating agent as well as in the reaction conditions, although the reaction takes place satisfactorily in suitable conditions. Likewise, the presence of a carbonyl group in said position 17 can also interfere and give rise to side reactions, although in this case good results are also achieved.

In addition, groups such as esters, ethers, acetals, etc., in position 17 do not seem to interact with the reagents used.

The starting products of general formula (II) can be obtained by known methods, for example from a Mannich type reaction of the suitable substrate, as mentioned in GB 2177700 or in EP 326340, describing how to obtain keto-derivatives in position 17, or by means of a process like that described by K. Annen in synthesis, (1982), page 34, through a reaction with phosphorus oxychloride, or according to the process described in Synthetic Communications, (1992), 22(16), pages 2305-12.

The compounds of general formula (II) with the 6-alkylidene group already introduced can be transformed into other compounds of general formula (II) by means of known methods, such as the acetylation of a hydroxyl group, or the oxidation thereof to a ketone and subsequent acetylation, etc., before being subjected to the dehydrogenation reaction.

An unsaturation is introduced in the 1,2 position of the compound of general formula (II) by means of the process of the invention, giving rise to the corresponding 1,4-diene derivative of general formula (I), wherein R represents H or C₁-C₆ alkyl, R₁ and R₂, independently of one another, represent H, OR₃, OC(=O)R₄, or O-(HPG), wherein R₃, R₄ and (HPG) have the previously indicated meanings; or R₁ and R₂, together with the carbon atom to which they are bonded, form a carbonyl group or equivalent or a cyclic ketal. In the event that the compound of general formula (I) is not the desired compound, it can be converted into the compound of general formula (I) by means of conventional methods.

In a particular embodiment, in the compound of general formula (I), R is H.

In another particular embodiment, in the compound of general formula (I), R is C₁-C₆ alkyl.

In another particular embodiment, in the compound of general formula (I), one of R₁ or R₂ is H and the other one is OR₃, wherein R₃ is H, C₁-C₆ alkyl, or aryl, preferably benzyl.

In another particular embodiment, in the compound of general formula (I), one of R₁ or R₂ is H and the other one is OC(=O)R₄, wherein R₄ is H, C₁-C₆ alkyl.

In another particular embodiment, in the compound of general formula (I), one of R₁ or R₂ is H and the other one is O-(HPG), wherein (HPG) is a hydroxyl protecting group having the previously indicated meaning.

In another particular embodiment, in the compound of general formula (I), R₁ and R₂ together with the carbon atom to which they are bonded, form a carbonyl group or equivalent or a cyclic ketal.

Particular preferred embodiments of the invention include obtaining compounds of general formula (I) wherein:
R is H; or
R₁ is H and R₂ is OH; or
R₁ is H and R₂ is OAc (wherein Ac is C(=O)CH₃); or
R₁ and R₂, together with the carbon atom to which they are bonded, form a carbonyl group (C=O).

In a particular preferred embodiment, the compound of general formula (I) obtained is 6-methylenandrost-1,4-dien-3,17-dione (exemestane).

The compound of general formula (I) obtained according to the process of the invention can be converted into another compound of general formula (I) by conventional methods, depending on the compound of general formula (I) obtained and on the compound of general formula (I) to be obtained.

In a particular embodiment, a compound of general formula (I), wherein one of R₁ or R₂ is H and the other one is OH, can be converted into a compound of general formula (I) wherein R₁ and R₂, together with the carbon atom to which they are bonded form a carbonyl group, by means of an oxidation reaction. Virtually any oxidation reaction and any reagent which allow oxidizing a hydroxyl group to a carbonyl group can be used; nevertheless, in a particular embodiment, said oxidation is carried out with a Cr(VI) reagent in acid medium, such as Jones reagent (aqueous solution of chromium trioxide and sulfuric acid) in the conditions described in EP 307134, for example. Therefore, in this case, the process of the invention first comprises subjecting the compound of general formula (II) wherein one of R₁ or R₂ is H and the other one is OH to a 1,2 dehydrogenation reaction in the previously described conditions (i.e., in the presence of a quinone, a silylating agent and a strong acid) to obtain a compound of general formula (I) wherein one of R₁ or R₂ is H and the other one is OH, and, secondly, converting said previously obtained compound of general formula (I) into a compound of general formula (I) wherein R₁ and R₂ together with the carbon atom to which they are bonded form a carbonyl group, by means of an oxidation reaction, as indicated above.

In another particular embodiment, a compound of general formula (I) wherein one of R₁ or R₂ is H and the other one is an (OC(=O)R₄ acyloxy group, wherein R₄ is C₁-C₆ alkyl), OAc for example, can be converted into a compound of general formula (I) wherein R₁ and R₂ together with the carbon atom to which they are bonded form a carbonyl group, by means of (i) transforming the acyloxy group (e.g., acetoxy (OAc)) into a hydroxyl group by conventional methods, for example, by means of saponification in basic medium using a suitable base, sodium hydroxide in methanol, for example, or, alternatively, by means of acid hydrolysis using a suitable acid, hydrochloric acid in a polar solvent, for example, and subsequently (ii) oxidizing the hydroxyl group to a carbonyl group, for example, by means of using a Cr(VI) reagent in acid medium, e.g., Jones reagent. Therefore, in this case, the process of the invention first comprises subjecting the compound of general formula (II) wherein one of R₁ or R₂ is H and the other one is an acyloxy group (e.g., OAc) to a 1,2 dehydrogenation reaction in the previously described conditions (i.e., in the presence of a quinone, a silylating agent and a strong acid) to obtain a compound of general formula (I) wherein one of R₁ or R₂ is H and the other one is an acyloxy group (e.g., OAc), and, secondly, converting said previously obtained compound of general formula (I) into a compound of general formula (I) wherein R₁ and R₂ together with the carbon atom to which they are bonded form a carbonyl group, by means of saponification or acid hydrolysis of the acid acyloxy group, followed by an oxidation reaction, as indicated above.

In another particular embodiment, the process of the invention comprises, prior to the dehydrogenation reaction, converting a compound of general formula (II) wherein one of R₁ or R₂ is H and the other one is OH into another compound of general formula (II) wherein one of R₁ or R₂ is H and the other one is an acyloxy group (e.g., OAc), such that the 1,2 dehydrogenation reaction is carried out on said compound of general formula (II) having an acyloxy group (e.g., OAc) in position 17, and, once said dehydrogenation reaction has ended, the compound of general formula (I) obtained (wherein one of R₁ or R₂ is H and the other one is an acyloxy group (e.g., OAc)) is converted into a compound of general formula (I), wherein one of R₁ or R₂ is H and the other one is OH, by conventional methods, for example, by means of acid or basic hydrolysis, and, if desired, said compound of general formula (I) having a hydroxyl group in position 17 is converted into another compound of general formula (I) wherein R₁ and R₂ together with the carbon atom to which they are bonded form a carbonyl group, by means of an oxidation reaction, as indicated above. The protection of the hydroxyl group by means of its derivatization into an acyloxy group before the dehydrogenation in the 1,2 position of the steroid allows significantly increasing the yield of the dehydrogenation reaction although said dehydrogenation reaction also occurs with good yields when the starting material has a hydroxyl group in position 17.

Other transformations allow obtaining a carbonyl group in position 17 of the steroid by means of the hydrolysis in acid medium of an ketal, once the 1,2 dehydrogenation reaction has been carried out, or by means of deprotecting a protected hydroxyl group in the form of an ester, an ether, e.g., a silyl ether, etc., by conventional methods, for example, by means of treatment with mineral acids, Lewis acids, organic sulfides, etc., depending on the nature of the hydroxyl protecting group, as described in the manual "Protective Groups in Organic Synthesis", 3rd Edition (1999), Green TW et al. for example, and subsequent oxidation of the derivative hydroxyl to the corresponding ketone by conventional methods by means of using a Cr(VI) reagent in acid medium, e.g., Jones reagent, for example. Merely by way of illustration, when the hydroxyl protecting group is a C₁-C₆ alkyl group, the removal of the hydroxyl protecting group can be carried out by treatment with aqueous hydrobromic acid in acetic acid, and optionally, in the presence of a phase transfer catalyst, such as an alkylammonium halide, tetrabutylammonium bromide, for example.

As mentioned above, in a particular preferred embodiment, the compound of general formula (I) obtained is 6-methylenandrost-1,4-dien-3,17-dione (exemestane). Therefore, in another aspect, the invention relates to a process for obtaining 6-methylenandrost-1,4-dien-3,17-dione (exemestane), comprising:
a) subjecting a compound of general formula (IIa) 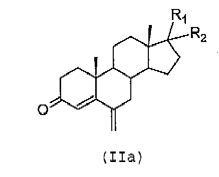 wherein
   R₁ and R₂, independently of one another, represent H, OR₃, OC(=O)R₄ or O-(HPG) wherein
   R₃is H, C₁-C₆ alkyl or aryl;
   R₄ is H or C₁-C₆ alkyl, and
   HPG is a hydroxyl protecting group; or
   R₁ and R₂, together with the carbon atom to which they are bonded, form a carbonyl group or equivalent or a cyclic ketal;
   to a dehydrogenation reaction in the 1,2 position in the presence of a quinone, a silylating agent and a strong acid, to obtain a compound of general formula (Ia) 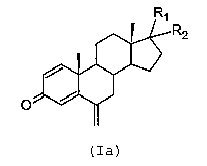 wherein R₁ and R₂ have the previously mentioned meanings; and
b) in the event that the compound of general formula (Ia) obtained does not have a carbonyl group (C=O) in position 17, converting said compound of general formula (Ia) into exemestane.

The conversion of a compound of general formula (Ia) which does not have a carbonyl group (C=O) in position 17, e.g., a compound of general formula (Ia) in which R₁ and R₂, independently of one another, represent H, OR₃, OC(=O)R₄ or O-(HPG), wherein R₃, R₄ and (HPG) have the previously mentioned meanings, or R₁ and R₂, together with the carbon atom to which they are bonded, form an equivalent group to carbonyl group (e.g., imine, oxime, hydrazone, etc.) or a cyclic ketal, can be carried out by conventional methods (e.g., by means of hydrolyzing and/or oxidizing the groups located in said position 17) known by the skilled person.

In a particular embodiment, the compound of general formula (II) used as starting material is a compound wherein R₁ and R₂ together with the carbon atom to which they are bonded form a carbonyl group (C=O), therefore the product obtained after carrying out step a) (dehydrogenation in the 1,2 position) is a compound of general formula (Ia) containing the carbonyl group in position 17, it is therefore not necessary to carry out additional conversions. In the event that the compound of general formula (IIa) used as starting material was a compound wherein R₁ and R₂ together with the carbon atom to which they are bonded form a carbonyl group (C=O) or a cyclic ketal, after the dehydrogenation reaction, a compound of general formula (Ia) containing a group equivalent to carbonyl or a cyclic acetal in position 17 would be obtained, it would therefore be necessary to carry out the corresponding conversions to obtain the carbonyl group in position 17. The necessary reagents and methods for carrying out such conversions are known by persons skilled in the art.

In another particular embodiment, the compound of general formula (IIa) used as starting material is a compound wherein one of R₁ or R₂ is H and the other one is an OC(=O)R₄ acyloxy group, wherein R₄ is H or C₁-C₆ alkyl (e.g., OAc), therefore the product obtained after carrying out step a) (dehydrogenation reaction in the 1,2 position) is a compound of general formula (Ia) containing an acyloxy group in position 17, it would therefore be necessary to convert said compound into exemestane. Briefly, the compound of general formula (Ia) obtained in step a) can be converted into a compound of general formula (Ia) wherein R₁ is OH and R₂ is H by means of hydrolysis in acid or basic medium by known methods (e.g., by means of saponification in basic medium using a suitable base, sodium hydroxide in methanol, for example, or by means of acid hydrolysis using a suitable acid, hydrochloric acid in a polar solvent, for example) and subsequent oxidation of the hydroxyl group to a carbonyl group, for example, by means of using a Cr(VI) reagent in acid medium, e.g., Jones reagent, as mentioned above, to obtain exemestane.

In another particular embodiment, the compound of general formula (IIa) used as starting material is a compound wherein one of R₁ or R₂ is H and the other one is OH, therefore the product obtained after carrying out step a) (dehydrogenation reaction in the 1,2 position) is a compound of general formula (Ia) containing a hydroxyl group in position 17, it would therefore be necessary to convert said compound into exemestane. Briefly, the compound of general formula (Ia) obtained in step a) can be converted into exemestane (compound of general formula (Ia) wherein R₁ and R₂, together with the carbon atom to which they are bonded form a carbonyl group by means of direct oxidation by known methods (e.g., by means of using a Cr(VI) reagent in acid medium, such as Jones reagent), as mentioned above.

Likewise, the compound of general formula (I) can form solvates by interaction with a solvent (including water, therefore the term "solvate" includes hydrates), such forms being included within the scope of the present invention.

The following examples illustrate the invention but must not be considered as limiting of the scope thereof.

### EXAMPLE 1

### Obtaining 6-methylenandrost-1,4-dien-3,17-dione

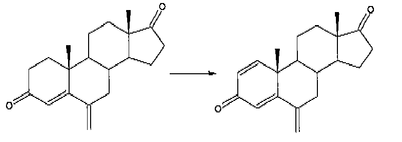

A mixture of 1 g of 6-methylenandrost-4-en-3,17-dione (3.5 mmol), 0.91 g of 2,3,5,6-tetrachloro-1,4-benzoquinone (chloranil) (3.7 mmol), 80 ml of toluene, 0.03 ml of trifluoromethanesulfonic acid (0.35 mmol) and 3.74 ml of bis(trimethylsilyl)trifluoroacetamide (BSTFA) (14.1 mmol) is stirred at reflux temperature (about 108-110°C) for 45 minutes. The mixture is then cooled at room temperature (20-22°C) and washed 6 times with a 2% aqueous sodium hydroxide solution (20 ml each time) and 3 times with a 30% aqueous sodium chloride solution. The organic phase is evaporated under reduced pressure, the solvent is substituted with heptane and the suspension is cooled. The product is filtered, washed with cold heptane and dried, obtaining 0.81 g [Yield : 81.8%] of crude 6-methylenandrost-1,4-dien-3,17-dione. The product can be purified by recrystallization in solvents or mixtures of solvents (ethyl acetate, ethyl acetate/heptane, ethanol or ethanol/water).

The recrystallized solid has a melting point (m.p.) of 191-194°C and the following spectroscopic characteristics:

¹H-NMR (DMSO-*d₆*) : 0.83 (3H, s, CH₃ 18), 1.09 (3H, s, CH₃ 19), 1.30-1.80 (10 H), 1.99 (1H, dt, J 9.6; 18.8 Hz, H16), 2.39 (1H, dd, J 8.8; 18.8 Hz, H16), 5.01 (2H, s, H 6a), 5.96 (1H, s, H4), 6.12 (1H, d, J 10.0 Hz, H2), 7.22 (1H, d, J 10.0 Hz, H1).

¹³C-NMR (DMSO-*d₆*): 14.1 (CH₃ 18), 20.1 (CH₃ 19), 22.0 (CH₂), 22.2 (CH₂), 31.5 (CH₂), 35.3 (CH), 35.9 (CH₂), 39.2 (CH₂), 44.1 (C 13), 47.7 (C 10), 49.9 (CH), 50.3 (CH), 112.8 (C 6 a), 122.4 (C4), 127.6 (C3), 146.1 (C5), 155.8 (C1), 168.2 (C6), 185.7 (C3), 219.7 (C17).

### EXAMPLE 2

### Obtaining 17β-hydroxy-6-methylenandrost-1,4-diene-3-one

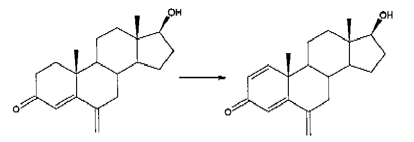

A mixture of 2 g of 17β-hydroxy-6-methylenandrost-4-en-3-one (7 mmol), 1.82 g of chloranil (7.4 mmol), 160 ml of toluene, 0.03 ml of trifluoromethanesulfonic acid (0.35 mmol) and 3.74 ml of BSTFA (14.1 mmol) is stirred at reflux temperature (about 108-110°C) for 4 hours. The mixture is then cooled at room temperature and washed 6 times with a 2% aqueous sodium hydroxide solution (20 ml each time) and 3 times with a 30% aqueous sodium chloride solution. The organic phase is distilled under vacuum, the solvent is substituted with heptane and the suspension is cooled. The product is filtered and washed with cold heptane, obtaining 1.36 g [Yield: 68.4%] of 17β-hydroxy-6-methylenandrost-1,4-diene-3-one. The product can be purified by recrystallization in solvents or mixtures of solvents (e.g., ethyl acetate, ethyl acetate/heptane, methanol or methanol/water).

The recrystallized solid has the following spectroscopic characteristics:

¹H-NMR (DMSO-*d₆*): 0.66 (3H, s, CH₃ 18), 1.06 (3H, s, CH₃ 19), 0.85-1.90 (12 H), 2.44 (1H, dd, J 3.6; 13.2 Hz), 3.41 (1H, m, H17), 4.51 (1H, s, OH), 4.96 (2H, s, H 6a), 5.94 (1H, s, H4), 6.12 (1H, dd, J 1.9, 10.2 Hz, H2), 7.22 (1H, d, J 10.2 Hz, H1).

¹³C-NMR (DMSO-*d₆*): 11.9, 20.2, 22.6, 23.7, 30.4, 36.0, 36.7, 43.2, 44.2, 50.2, 50.5, 80.3, 112.5, 122.3, 127.5, 146.5, 156.0, 168.5, 185.8.

### EXAMPLE 3

### Obtaining 17β-acetoxy-6-methylenandrost-1,4-diene-3-one

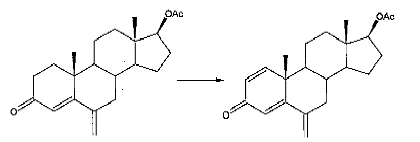

### 3.1 Catalyzed by sulfuric acid

A mixture of 10 grams of 17β-acetoxy-6-methylenandrost-4-en-3-one (29 mmol), 8 g of chloranil (33 mmol), 300 ml of toluene, 0.8 ml of sulfuric acid (14.5 mmol) and 25 ml of BSTFA (94 mmol) is stirred at reflux temperature (about 108-110°C) for 15 hours. The mixture is then cooled at room temperature and washed with a 5% aqueous sodium metabisulfite solution (50 ml), 3 times with a 2% aqueous sodium hydroxide solution (100 ml each time) and 3 times with a 30% aqueous sodium chloride solution (100 ml each time). The solvent is eliminated and the residue is purified by silica gel column chromatography using mixtures of ethyl acetate/heptane as an eluent to obtain 8.0 g [Yield: 80.2%] of 17β-acetoxy-6-methylenandrost-1,4-diene-3-one.

The recrystallized solid has the following spectroscopic characteristics:
¹H-NMR (DMSO-*d₆*): 0.79 (3H, s, CH₃ 18), 1.20 (3H, s, CH₃ 19), 1.00-1.90, 1.97 (3H, s, CH₃ Ac), 2.00-2.10, 2.47, 4.50 (1H, H17), 4.98 (2H, s), 5.95 (1H, s, H4), 6.12 (1H, dd, J 1.9, 10.2 Hz, H2), 7.20 (1H, d, J 10.2 Hz, H1).
¹³C-NMR (DMSO-*d₆*): 12.6, 20.2, 21.5, 22.5, 23.7, 27.7, 35.6, 36.6, 40.0, 42.9, 44.1, 49.9, 50.0, 82.2, 112.6, 122.3, 127.6, 146.3, 155.8, 168.3, 170.9, 185.7.

### 3.2 Catalyzed by trifluoromethanesulfonic acid

3 g of 17β-acetoxy-6-methylenandrost-4-en-3-one (8.7 mmol), 2.4 g of chloranil (10 mmol), 90 ml of toluene, 0.09 ml of trifluoromethanesulfonic acid (0.87 mmol) and 7.5 ml of BSTFA (28.2 mmol) are stirred at reflux temperature (about 108-110°C) for 30 minutes. The mixture is then cooled at room temperature and washed with a 5% aqueous sodium metabisulfite solution (15 ml), 3 times with a 2% aqueous sodium hydroxide solution (30 ml each time) and 3 times with a 30% aqueous sodium chloride solution (30 ml each time). The solvent is eliminated and the residue is purified by silica gel column chromatography using mixtures of ethyl acetate/heptane as an eluent to obtain 2.55 g [Yield: 85.3%] of 17β-acetoxy-6-methylenandrost-1,4-diene-3-one.

### 3.3 Catalyzed by p-toluenesulfonic acid

A solution of 1 g of 17β-acetoxy-6-methylenandrost-4-en-3-one (3.5 mmol), 0.8 g of chloranil (3.7 mmol), 30 ml of toluene, 0.02 g of p-toluenesulfonic acid (0.1 mmol) and 2.85 ml of BSTFA (10.8 mmol) is stirred at reflux temperature (about 108-110°C) for 72 hours. The mixture is then cooled at room temperature and washed with a 5% aqueous sodium metabisulfite solution (10 ml), 3 times with a 2% aqueous sodium hydroxide solution (20 ml each time) and 3 times with a 30% aqueous sodium chloride solution. The solvent is eliminated and the residue is purified by column chromatography using mixtures of ethyl acetate/heptane as an eluent to obtain 0.66 g [Yield: 66.6%] of 17β-acetoxy-6-methylenandrost-1,4-diene-3-one.

### 3.4 Catalyzed by trifluoroacetic acid

A mixture of 0.3 g of 17β-acetoxy-6-methylenandrost-4-en-3-one (1 mmol), 0.24 g of chloranil (1.1 mmol), 9 ml of toluene, 0.05 ml of trifluoroacetic acid (0.2 mmol) and 1.2 ml of BSTFA (4.4 mmol) is stirred at reflux temperature (about 108-110°C) for 48 hours. The mixture is then cooled at room temperature and washed with a 5% aqueous sodium metabisulfite solution (1.5 ml), 3 times with a 2% aqueous sodium hydroxide solution (3 ml each time) and 3 times with a 30% aqueous sodium chloride solution (3 ml each time). The solvent is eliminated and the residue is purified by column chromatography using mixtures of ethyl acetate/heptane as an eluent to obtain 0.2 g [Yield: 67.3%] of 17β-acetoxy-6-methylenandrost-1,4-diene-3-one.

### 3.5 Catalyzed by trifluoroacetic acid and using 2,3-dichloro-5,6-dicyanobenzoquinone (DDQ) as a dehydrogenating agent

A mixture of 2 g of 17β-acetoxy-6-methylenandrost-4-en-3-one (7 mmol), 1.76 g of 2,3-dichloro-5,6-dicyanobenzoquinone (DDQ) (7.4 mmol), 60 ml of toluene, 0.1 ml of trifluoroacetic acid (1.4 mmol) and 8 ml of BSTFA (29.4 mmol) is stirred at 60°C for 32 hours. The mixture is then cooled at room temperature and washed with a 5% aqueous sodium metabisulfite solution (10 ml), 3 times with a 2% aqueous sodium hydroxide solution (20 ml each time) and 3 times with a 30% aqueous sodium chloride solution (20 ml each time). The solvent is eliminated and the residue is purified by column chromatography using mixtures of ethyl acetate/heptane as an eluent to obtain 0.49 g [Yield: 24.7%] of 17β-acetoxy-6-methylenandrost-1,4-diene-3-one.

### 3.6 Catalyzed by trifluoromethanesulfonic acid and using 2,3-dichloro-5,6-dicyanobenzoquinone as a dehydrogenating agent

A mixture of 0.5 g of 17β-acetoxy-6-methylenandrost-4-en-3-one (1.8 mmol), 0.44 g of 2,3-dichloro-5,6-dicyanobenzoquinone (1.85 mmol), 15 ml of toluene, 0.02 ml of trifluoromethanesulfonic acid (0.18 mmol) and 2 ml of BSTFA (7.2 mmol) is stirred at 20°C for 48 hours. The mixture is then washed with a 5% aqueous sodium metabisulfite solution (2.5 ml), 3 times with a 2% aqueous sodium hydroxide solution (5 ml each time) and 3 times with a 30% aqueous sodium chloride solution (5 ml each time). The solvent is eliminated and the residue is purified by column chromatography using mixtures of ethyl acetate/heptane as an eluent to obtain 0.25 g [Yield: 50.5%] of 17β-acetoxy-6-methylenandrost-1,4-diene-3-one.

### 3.7 Using trimethylsilylimidazole as a silylating agent

A mixture of 0.5 g of 17β-acetoxy-6-methylenandrost-4-en-3-one (1.5 mmol), 0.4 g of chloranil (1.7 mmol), 15 ml of toluene, 0.02 ml of trifluoromethanesulfonic acid (0.15 mmol) and 1.8 ml of trimethylsilylimidazole (5.9 mmol) is stirred under toluene reflux (about 108-110°C) for 53 hours. The mixture is then cooled at room temperature and washed with a 5% aqueous sodium metabisulfite solution (2.5 ml), 3 times with a 2% aqueous sodium hydroxide solution (5 ml each time) and 3 times with a 30% aqueous sodium chloride solution (5 ml each time). The solvent is eliminated and the residue is purified by column chromatography using mixtures of ethyl acetate/heptane as an eluent to obtain 0.02 g [Yield: 4.04%] of 17β-acetoxy-6-methylenandrost-1,4-diene-3-one.

### 3.8 Using trimethylsilylurea as a silylating agent

A mixture of 0.3 g of 17β-acetoxy-6-methylenandrost-4-en-3-one (0.9 mmol), 0.25 g of chloranil (1 mmol), 9 ml of toluene, 0.01 ml of trifluoromethanesulfonic acid (0.09 mmol) and 0.75 g of trimethylsilylurea (3.5 mmol) is stirred under toluene reflux (about 108-110°C) for 68 hours. The mixture is then cooled at room temperature and washed with a 5% aqueous sodium metabisulfite solution (2.5 ml), 3 times with a 2% aqueous sodium hydroxide solution (5 ml each time) and 3 times with a 30% aqueous sodium chloride solution (5 ml each time). The solvent is eliminated and the residue is purified by column chromatography using mixtures of ethyl acetate/heptane as an eluent to obtain 0.05 g [Yield: 16.8%] of 17β-acetoxy-6-methylenandrost-1,4-diene-3-one.

### EXAMPLE4 4

### Transformation of 17β-acetoxy-6-methylenandrost-1,4-diene-3-one into 6-methlene-17β-hdroxyandrost-1,4-diene-3-one and into 6-inethylenandrost-1,4-dien-3,17-dione (Exemestane)

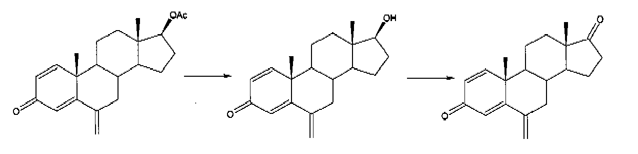

A solution of 0.86 kg (21.5 mol) of sodium hydroxide in 32 1 of methanol is loaded on a suspension of 7.15 kg (21.5 mol) of 17β-acetoxy-6-methylenandrost-1,4-diene-3-one (Example 3) in 21 1 of methanol. It is stirred for 2 hours and the mixture is neutralized with 1.3 kg (21.5 mol) of acetic acid. The product crystallizes by the addition of 57 1 of water, the suspension is cooled, filtered and washed to obtain 6.25 kg of 6-methylene-17β-hydroxyandrost-1,4-diene-3-one.

6.0 kg (20.9 mol) of 6-methylene-17β-hydroxyandrost-1,4-diene-3-one in the form of hydrate are suspended in 120 ml of acetone at 0°C and a solution of Jones reagent prepared with 2.46 kg (24.6 mol) of chromium trioxide, 7.2 1 of water and 2.1 1 of sulfuric acid is slowly loaded, checking the persistent red color of the reaction mixture. 3 1 of isopropanol are added, the chromium salts are filtered, the acetone is distilled up to 15% of the initial volume and the product crystallizes by the addition of water (48 1), filtration and drying. 4.80 kg of 6-methylenandrost-1,4-dien-3,17-dione (exemestane) are obtained. The exemestane can be purified by recrystallization in solvents or mixtures of solvents (ethyl acetate, ethyl acetate/heptane, ethanol or ethanol/water).

### EXAMPLE 5 (reference example)

### Reaction of 17β-hydroxy-6-methylenandrost-4-en-3-one with DDQ and dioxane

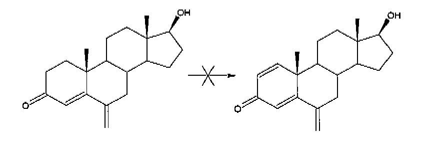

0.27 g (0.96 mmol) of 17β-hydroxy-6-methylene-androst-4-en-3-one and 0.34 g (1,50 mmol) of 2,3-diohloro-5,6-dicyanobenzoquinone (DDQ) in 20 ml of dioxane are stirred at reflux temperature (about 100-102°C) in an inert atmosphere for 18 hours. The reaction mixture is filtered on alumina, it is washed with dioxane, the solvent is eliminated under reduced pressure and the residue is filtered through an alumina bed eluting with ethyl acetate. The solution is washed with water, dried with sodium sulfate and the solvent is evaporated, obtaining 0.0458 g of a crude reaction product in which the expected product 6-methylene-17β-hydroxyandrost-1,4-diene-3-one is not detected.

### EXAMPLE 6 (reference example)

### Reaction of 6-methylenandrost-4-en-3,17-dione with DDQ and dioxane

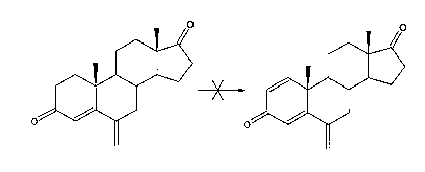

5 g (0.017 mol) of 6-methylene-androst-4-en-3,17-di-one and 5.7 g (0.025 mol) of 2,3-dichloro-5,6-dicyanobenzoquinone (DDQ) in 200 ml of dioxane are stirred at reflux temperature (about 100-102°C) in an inert atmosphere for 15 hours. The reaction mixture is filtered on alumina, it is washed with dioxane, the solvent is eliminated under reduced pressure. The residue is redissolved in ethyl acetate, it is washed with water, dried with sodium sulfate and the solvent is evaporated, obtaining 3.5 g of a crude reaction product in which the expected product 6-methylenandrost-1,4-diene-3,17-dione (exemestane) is not detected.

### EXAMPLE 7 (reference example)

### Reaction of 17β-hydroxy-6-methylenandrost-4-en-3-one with DDQ in the presence of benzoic acid

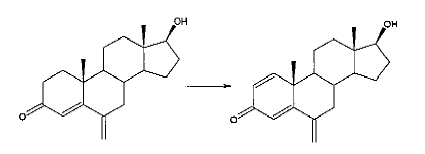

0.5 g (1.66 mmol) of 17β-hydroxy-6-methylenandrost-4-en-3-one, 0.57 g (2.5 mmol) of 2,3-dichloro-5,6-dicyanobenzoquinone (DDQ) and 0.1 g (2.33 mmol) of benzoic acid in 20 ml of benzene are stirred at reflux temperature (about 80-82°C) in an inert atmosphere for 5 hours. The reaction mixture is purified by silica gel column chromatography, obtaining 0.07 g of 17β-hydroxy-6-methylenanorost-1,4-dien-3-one (Yield: 14%).

## Claims

1. A process for obtaining a 6-alkylidenandrost-1,4-dien-3-one of general formula (I) 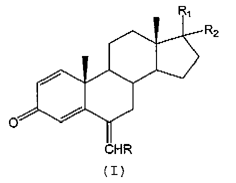 wherein
R is H or C₁-C₆ alkyl;
R₁ and R₂, independently of one another, represent H, OR₃, OC(=O)R₄ or O-(HPG), wherein
R₃ is H, C₁-C₆ alkyl or aryl;
R₄ is H or C₁-C₆ alkyl, and
HPG is a hydroxyl protecting group; or
R₁ and R₂, together with the carbon atom to which they are bonded, form a carbonyl group or equivalent or a cyclic ketal;
and its solvates,
comprising
a) subjecting a compound of general formula (II) 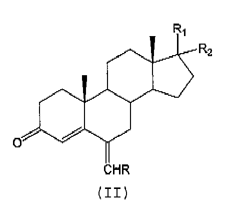 wherein R, R₁ and R₂ have the previously mentioned meanings;
to a dehydrogenation reaction in the 1,2 position in the presence of a quinone, a silylating agent and a strong acid; and, if desired,
b) converting a compound of general formula (I) into another compound of general formula (I).

2. A process according to claim 1, wherein said silylating agent is selected from the group consisting of a bistrimethylsilyltrihaloacetamide, bistrimethylsilylacetamide, hexamethyldisilazane, bistrimethylsilylurea and mixtures thereof.

3. A process according to claim 2, wherein said silylating agent is bistrimethylsilyltrifluoroacetamide (BSTFA).

4. A process according to claim 1, wherein said quinone is selected from the group consisting of 2,3,5,6-tetrachloro-1,4-benzoquinone, 2,3-dichloro-5,6-dicyanobenzoquinone and mixtures thereof.

5. A process according to claim 4, wherein said quinone is 2,3,5,6-tetrachloro-1,4-benzoquinone.

6. A process according to claim 1, wherein said strong acid is selected from the group consisting of trifluoroacetic acid, p-toluenesulfonic acid, methanesulfonic acid, trifluoromethanesulfonic acid, sulfuric acid, hydrobromic acid, perchloric acid and mixtures thereof.

7. A process according to claim 6, wherein said strong acid is selected from trifluoroacetic acid, trifluoromethanesulfonic acid, sulfuric acid and mixtures thereof.

8. A process according to claim 1, wherein said quinone is 2,3,5,6-tetrachloro-1,4-benzoquinone, the silylating agent is bistrimethylsilyltrifluoroacetamide, and the strong acid is trifluoromethanesulfonic acid or sulfuric acid.

9. A process according to claim 1, which comprises converting a compound of general formula (I) into another compound of general formula (I) by means of hydrolyzing and/or oxidizing the group present in position 17.

10. A process according to claim 9, which comprises converting a compound of general formula (I) wherein one of R₁ or R₂ is H and the other one is OH into a compound of general formula (I) wherein R₁ and R₂ together with the carbon atom to which they are bonded, form a carbonyl group by means of an oxidation reaction of the hydroxyl group.

11. A process according to claim 10, wherein the oxidation of the group is carried out with a Cr(VI) reagent in acid medium.

12. A process according to claim 9, which comprises converting a compound of general formula (I) wherein one of R₁ or R₂ is H and the other one is an acyloxy group (OC(=O)R₄), wherein R₄ has the previously indicated meaning into a compound of general formula (I) wherein R₁ and R₂ together with the carbon atom to which they are bonded form a carbonyl group, by means of hydrolyzing the acyloxy group and subsequently oxidizing the generated hydroxyl group.

13. A process for obtaining 6-methylenandrost-1,4-dien-3,17-dione (exemestane) comprising:
a) subjecting a compound of general formula (IIa) 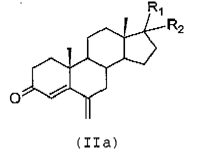 wherein R₁ and R₂, independently of one another, represent
H, OR₃, OC(=O)R₄ or O-(HPG), wherein
R₃ is H, C₁-C₆ alkyl or aryl;
R₁ is H or C₁-C₆ alkyl, and
HPG is a hydroxyl protecting group; or
R₁ and R₂, together with the carbon atom to which they are bonded, form a carbonyl group or equivalent or a cyclic ketal;
to a dehydrogenation reaction in the 1,2 position in the presence of a quinone, a silylating agent and a strong acid; to obtain a compound of general formula (Ia) 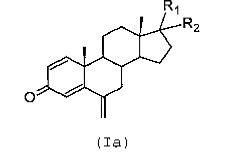 wherein R₁ and R₂ have the previously mentioned meanings;
b) in the event that the compound of general formula (Ia) obtained does not have a carbonyl group (C=O) in position 17, converting said compound of general formula (Ia) into exemestane.

14. A process according to claim 13, wherein the compound of general formula (IIa) is a compound wherein R₁ and R₂ together with the carbon atom to which they are bonded form a carbonyl group.

15. A process according to claim 13, wherein the compound of general formula (IIa) is a compound wherein one of R₁ or R₂ is H and the other one is an OC(=O)R₄ acyloxy group, wherein R₄ has the previously indicated meanings, said process comprising the conversion of the compound of general formula (Ia) obtained in step a), containing an acyloxy group in position 17, into exemestane by means of hydrolyzing the acyloxy group and subsequently oxidizing the generated hydroxyl group.

16. A process according to claim 13, wherein the compound of general formula (IIa) is a compound wherein one of P₁ or R₂ is H and the other one is OH, said process comprising the oxidation of the hydroxyl group of the compound of general formula (Ia) obtained in step a).

17. A process according to any of claims 15 or 16, wherein the oxidation of the hydroxyl group is carried out with a Cr(VI) reagent in acid medium.
